# EUROPÄISCHE PATENTANMELDUNG

(11) **EP 1 167 325 A1**
(43) Veröffentlichungstag der Anmeldung: **02.01.2002**
(21) Anmeldenummer: 01109385.3
(22) Anmeldetag: 19.04.2001
(51) Int. Cl.: C07B 41/02, C07B 43/04, C07C 67/343, C07C 69/675, C07D 205/08

(54) **Verfahren zur Herstellung von Hydroxy- und Aminoverbindungen**

(30) Priorität: 29.06.2000 DE 10031604
(71) Anmelder: Consortium für elektrochemische Industrie GmbH, 81379 München (DE)
(72) Erfinder: Sorger, Klaus, Dr., 81371 München (DE); Petersen, Hermann, Dr., 84489 Burghausen (DE)
(74) Vertreter: Fritz, Helmut, Dr.

(57) **Zusammenfassung**

Verfahren zur Herstellung von Hydroxy- und Aminoverbindungen, bei dem in einem ersten Schritt Aldehyde, Ketone und Imine in einer Reformatsky-Reaktion mit einer reaktiven Halogenverbindung und Zink in Carbonsäureestern als Lösungsmittel umgesetzt werden, wobei die reaktive Halogenverbindung gleichzeitig mit oder nach den Aldehyden, Ketonen und Iminen mit dem Zink in Kontakt gebracht wird und bei dem in einem zweiten Schritt das Reaktionsprodukt des ersten Schritts hydrolysiert wird.

## Beschreibung

Die Erfindung betrifft ein Verfahren zur Herstellung von Hydroxy- und Aminoverbindungen.

Die Umsetzung von reaktiven Halogenverbindungen, insbesondere α-Halogencarbonylverbindungen mit elektrophilen Substraten wie z. B. Aldehyden, Ketonen, Iminen, Nitrilen, Carbonsäureanhydriden, -chloriden, Lactonen, Orthoformiaten, Formiaten, Epoxiden, Azirinen, Aminalen und Nitronen in Gegenwart von Zinkmetall, bekannt als Reformatsky-Reaktion, liefert wichtige Synthesebausteine für die Herstellung von pharmazeutischen Wirkstoffen, Duftstoffen und Pflanzenschutzmitteln.
Die Wahl des Lösungsmittels und die Aktivierung des eingesetzten Zinks bzw. der gesamten Reaktionsmischung sind zur Erzielung guter Ausbeuten und hoher Selektivitäten und damit hoher Produktreinheiten von entscheidender Bedeutung.

Es ist bekannt, daß als Lösungsmittel für die Reformatsky-Reaktion Ether wie Diethylether, 1,4-Dioxan, Dimethoxymethan, Dimethoxyethan und insbesondere Tetrahydrofuran besonders geeignet sind. Daneben haben sich als Lösungsmittel auch aromatische Kohlenwasserstoffe oder Gemische der vorstehend genannten Ether mit aromatischen Kohlenwasserstoffen, die Mischung von Tetrahydrofuran und Borsäuretrimethylester oder die polaren Lösungsmittel Acetonitril, Dimethylformamid, Dimethylacetamid, Dimethylsulfoxid und Hexamethylphosphorsäuretriamid bewährt. Eine zusammenfassende Darstellung hierzu ist beispielsweise in A. Fürstner, Synthesis 1989, 571 enthalten.
Aus EP-A-562 343 ist bekannt, daß die Umsetzung von α-Bromcarbonsäureestern mit Carbonylverbindungen in Gegenwart von Zink in dem Lösungsmittel Methylenchlorid mit hohen Ausbeuten verläuft.

Die Verwendung der genannten Lösungsmittel bzw. Lösungsmittelgemische hat folgende Nachteile:
Die mit Wasser mischbaren Ether 1,4-Dioxan und Tetrahydrofuran sowie die mit Wasser mischbaren polaren Lösungsmittel Acetonitril, Dimethylformamid, Dimethylacetamid, Dimethylsulfoxid und Hexamethylphosphorsäuretriamid lösen sich bei wäßriger Aufarbeitung zur Hydrolyse der gebildeten Zinkverbindungen und Zinksalze in der wäßrigen Phase. Aus Gründen der Wirtschaftlichkeit und zur Verringerung der Abfallmengen ist insbesondere bei Anwendung im technischen Maßstab eine Rückgewinnung der eingesetzten Lösungsmittel aus der wäßrigen Phase, z. B. durch Extraktion oder Destillation erforderlich, was allerdings mit erheblichem Aufwand verbunden ist.

Zudem ist bei Verwendung der vorstehend genannten, mit Wasser mischbaren Lösungsmittel bei Hydrolyse des Reaktionsgemisches üblicherweise der Einsatz von mit Wasser nicht mischbaren organischen Lösungsmitteln wie Ethylacetat oder Methyl-tert.-butylether als Cosolvenzien zur besseren Phasentrennung notwendig. Diese Lösungsmittel müssen zurückgewonnen und vor Wiederverwendung durch Destillation von Verunreinigungen befreit werden, was ebenfalls mit hohem Aufwand verbunden ist. Bei Einsatz von Lösungsmittelgemischen für Reformatsky-Reaktionen erfordert die Rückgewinnung, Trennung und gegebenenfalls Reinigung der einzelnen eingesetzten Lösungsmittel generell erheblichen Mehraufwand.

Ferner neigen Diethylether, 1,4-Dioxan, Dimethoxymethan, Dimethoxyethan und Tetrahydrofuran als Lösungsmittel für Reformatsky-Reaktionen durch Autoxidation zur Bildung von explosiven Peroxiden, was deren Einsatz im technischen Maßstab gefährlich macht, insbesondere bei wiederholter Verwendung nach Rückgewinnung (Gefahr der Anreicherung der explosiven Bestandteile), erschwert oder nur unter großem Aufwand möglich macht.

Die Verwendung von Methylenchlorid als Lösungsmittel, wie aus EP-A-562 343 bekannt, oder anderen halogenierten Kohlenwasserstoffen als Lösungsmittel ist aus Umweltschutzgründen bedenklich und deshalb insbesondere im technischen Maßstab zu vermeiden. Zudem sind viele der vorstehend genannten Lösungsmittel teuer, was die Wirtschaftlichkeit der Reaktion ohne Rückgewinnung des eingesetzten Lösungsmittels zusätzlich beeinträchtigt.

Es bestand daher immer noch ein Bedürfnis nach einem geeigneten Lösungsmittel für die Umsetzung von Aldehyden, Ketonen und Iminen mit reaktiven Halogenverbindungen und Zink, das es erlaubt, die Reaktion insbesondere im technischen Maßstab ohne Verwendung von Lösungsmittelgemischen oder Zusatz von Cosolvenzien bei möglichst einfacher Rückgewinnung des eingesetzten Lösungsmittels und damit unter Reduktion der Abfallmengen umweltfreundlich und kostengünstig durchzuführen, ohne dabei die Ausbeute und Selektivität im Vergleich zu den vorstehend genannten, vorbekannten Lösungsmitteln zu mindern.

Gegenstand der Erfindung ist ein Verfahren zur Herstellung von Hydroxy- und Amino-verbindungen, bei dem
in einem ersten Schritt Aldehyde, Ketone und Imine in einer Reformatsky-Reaktion mit einer reaktiven Halogenverbindung und Zink in Carbonsäureestern als Lösungsmittel umgesetzt werden, wobei die reaktive Halogenverbindung gleichzeitig mit oder nach den Aldehyden, Ketonen und Iminen mit dem Zink in Kontakt gebracht wird
und bei dem in einem zweiten Schritt das Reaktionsprodukt des ersten Schritts hydrolysiert wird.

Bevorzugt handelt es sich um ein Verfahren zur Herstellung von Hydroxy- und Aminoverbindungen der allgemeinen Formel (1)

R¹R²C(W^{x})-R³R⁴C-(X)₁-Y (1),

bei dem
in einem ersten Schritt Aldehyde, Ketone und Imine der allgemeinen Formel (2)

R¹R²C=W (2)

mit reaktiven Halogenverbindungen der allgemeinen Formel (3)

Hal-R³R⁴C-(X)₁-Y (3),

und Zink in Carbonsäureestern der allgemeinen Formel (4) als Lösungsmittel

R⁵-((O(CH₂)ₘ)ₙ-COO-((CH₂)ₒ-COO)ₚ-((CH₂)_{q}O)ᵣ-R⁶ (4),

umgesetzt werden, wobei
**R**^{**1**} und **R**^{**2**} Wasserstoff oder einen gegebenenfalls halogen- oder cyanosubstituierten C₁-C₃₀-Kohlenwasserstoffrest, in dem eine oder mehrere, einander nicht benachbarte Methyleneinheiten durch Gruppen -O-, -CO-, -COO-, -OCO-, oder -OCOO-, -S- oder -N**R**^{**x**}- ersetzt sein können und in dem eine oder mehrere Methineinheiten durch Gruppen -N= oder - P= ersetzt sein können,
**W**^{**x**} OH oder NHR¹,
**W** O oder NR¹,
**R**^{**3**} und **R**^{**4**} Wasserstoff, Halogen oder einen gegebenenfalls halogensubstituierten C₁-C₃₀-Kohlenwasserstoffrest, in dem eine oder mehrere, einander nicht benachbarte Methyleneinheiten durch Gruppen -O-, -CO-, -COO-, -OCO-, oder -OCOO-, -S- oder -N**R**^{**x**}- ersetzt sein können und in dem eine oder mehrere Methineinheiten durch Gruppen -N= oder - P= ersetzt sein können,
**X** ausgewählt wird aus 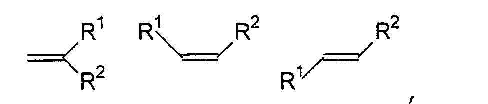
**1** eine ganze Zahl im Wert von 0 oder 1,
**Y** CN, (C=O)-Z, (SO₂)-Z, (P=O)(-Z)₂ oder ein aromatischer Rest, wobei eine oder mehrere Methineinheiten im Ring durch Gruppen -N= oder -P= ersetzt sein können und der im Ring die Heteroatome -O-, -S- oder -NH- tragen kann, wobei der aromatische Ring gegebenenfalls halogen- oder cyanosubstituiert oder durch C₁-C₃₀-Kohlenwasserstoffreste, in denen eine oder mehrere, einander nicht benachbarte Methyleneinheiten durch Gruppen -O-, -CO-, -COO-, -OCO-, oder -OCOO-, -S- oder -N**R**^{**x**}- ersetzt sein können, substituiert ist,
**Z** einen gegebenenfalls halogensubstituierten C₁-C₃₀-Kohlenwasserstoffrest, in dem eine oder mehrere, einander nicht benachbarte Methyleneinheiten durch Gruppen -O-, -CO-, -COO-, -OCO-, oder -OCOO-, -S-, oder -N**R**^{**x**}- ersetzt sein können und in dem eine oder mehrere Methineinheiten durch Gruppen -N= oder -P= ersetzt sein können, OH, OR¹, OSi(R¹)₃, NHR¹ oder NR¹R²,
**Hal** Chlor, Brom oder Iod,
**R**^{**5**} und **R**^{**6**} einen C₁-C₃₀-Kohlenwasserstoffrest, in dem eine oder mehrere, einander nicht benachbarte Methyleneinheiten durch Gruppen -O- ersetzt sein können,
**R**^{**x**} Wasserstoff oder einen gegebenenfalls halogensubstituierten C₁-C₃₀-Kohlenwasserstoffrest, in dem eine oder mehrere, einander nicht benachbarte Methyleneinheiten durch Gruppen -O-, -CO-, -COO-, -OCO-, oder -OCOO-, -S-, -NH- oder -NCH₃-ersetzt sein können und in dem eine oder mehrere Methineinheiten durch Gruppen -N= oder -P= ersetzt sein können,
**m, n, o, p, q,** und **r** ganze Zahlen im Wert von 0 bis 6 bedeuten, wobei jeweils 2 Reste, die ausgewählt werden aus den Paaren **R**^{**1**} und **R**^{**2**}**, R**^{**3**} und **R**^{**4**}**, R**^{**1**} und **R**^{**3**}**, R**^{**1**} und **Y, R**^{**1**} und **Z, R**^{**3**} und **Y, R**^{**3**} und **Z, W**^{**x**} und **Y** sowie **W**^{**x**} und **Z,** wobei **W**^{**x**} O- oder NR¹- bedeutet und **Z** eine direkte Bindung bedeuten kann, miteinander verknüpft sein können,
wobei die reaktiven Halogenverbindungen der allgemeinen Formel (3) gleichzeitig mit oder nach den Verbindungen der allgemeinen Formel (2) mit dem Zink in Kontakt gebracht werden und
bei dem in einem zweiten Schritt das Reaktionsprodukt des ersten Schritts hydrolysiert wird.

Nach dem vorstehend beschriebenen, erfindungsgemäßen Verfahren lassen sich Hydroxy- und Aminoverbindungen der allgemeinen Formel (1) in sehr hohen Ausbeuten von bis zu > 90 % und sehr hohen Reinheiten einfach und bei gleichzeitig sehr guten Raum-Zeit-Ausbeuten gewinnen.

Das erfindungsgemäße Verfahren gestaltet sich besonders im technischen Maßstab einfach, da die Reaktion in den kommerziell erhältlichen Carbonsäureestern der allgemeinen Formel (4) als Lösungsmittel durchgeführt werden kann, ohne daß eine Vorbehandlung dieser Lösungsmittel wie z. B. Destillation oder Trocknung notwendig und weder für die Durchführung der Reaktion noch für die Aufarbeitung der Zusatz eines weiteren Lösungsmittels wie z. B. eines Cosolvens zur besseren Phasentrennung bei Aufarbeitung erforderlich ist. Da keine Lösungsmittelgemische eingesetzt werden müssen, gestaltet sich die Rückgewinnung der verwendeten Lösungsmittel sehr einfach. Die bevorzugt verwendeten Carbonsäureester der allgemeinen Formel (4) lösen sich wenig oder nur sehr geringfügig in Wasser und können somit beispielsweise bei der Produktisolierung leicht und effizient zurückgewonnen werden, was die Reaktion sehr wirtschaftlich macht. So läßt sich z. B. Ethylacetat, Isopropylacetat oder Butylacetat als Lösungsmittel der allgemeinen Formel (4) bei der Isolierung der hergestellten Produkte durch Destillation in sehr hohen Ausbeuten zurückgewinnen.

Das erfindungsgemäße Verfahren ermöglicht zudem den Ersatz von halogenhaltigen Kohlenwasserstoffen und gegenüber Peroxidbildung empfindlichen Ethern durch Carbonsäureester der allgemeinen Formel (4) als Lösungsmittel, wodurch das erfindungsgemäße Verfahren nicht nur umweltfreundlicher sondern auch mit einem deutlich reduzierten Gefahrenpotenzial verbunden ist. Ferner ist die Produktausbeute und -qualität der nach der erfindungsgemäßen Verfahrensvariante hergestellten Hydroxy- und Aminoverbindungen der allgemeinen Formel (1) gegenüber vorbekannten, in der Literatur beschriebenen Varianten in vielen Fällen erhöht. Die Verwendung von Carbonsäureestern der allgemeinen Formel (4) ist für den Verlauf der Reaktion, die Produktausbeuten und -reinheiten und die Eliminierung von Nebenreaktionen von besonderem Vorteil.
Zudem kann beim erfindungsgemäßen Verfahren in den meisten Fällen mit einem geringeren Überschuß an Zink und reaktiver Halogenverbindung der allgemeinen Formel (3), bezogen auf den elektrophilen Reaktionspartner, gearbeitet werden, wobei die Produktausbeute und -qualität in vielen Fällen höher ist als bei vorbekannten Varianten unter Verwendung der erwähnten, vorbekannten Lösungsmittel, was das erfindungsgemäße Verfahren besonders wirtschaftlich macht.

Beim erfindungsgemäßen Verfahren werden vorzugsweise zuerst Zink und Aldehyd, Keton oder Imin der allgemeinen Formel (2) in dem Carbonsäureester der allgemeinen Formel (4) vorgelegt und anschließend die reaktive Halogenverbindung der allgemeinen Formel (3) zugegeben, gegebenenfalls gelöst in einem Lösungsmittel.
Ebenfalls bevorzugt wird zuerst Zink in dem Carbonsäureester der allgemeinen Formel (4) vorgelegt und dann eine Mischung aus reaktiver Halogenverbindung der allgemeinen Formel (3) und Aldehyd, Keton oder Imin der allgemeinen Formel (2) zugeben, gegebenenfalls gelöst in einem Lösungsmittel.

Aus E. W. Warnhoff, M. Y. H. Wong, P. S. Raman, Can. J. Chem. 1981, 59, 688 ist bekannt, daß α-Halogenozinkcarbonylverbindungen (Reformatsky-Reagenzien) der allgemeinen Formel (3), wobei Hal Halogenozink bedeutet, mit Carbonsäureestern als Elektrophile reagieren können. Da Aldehyde, Ketone und Imine elektrophiler und damit reaktiver als Carbonsäurederivate wie die im erfindungsgemäßen Verfahren als Lösungsmittel verwendeten Carbonsäureester der allgemeinen Formel (4) sind, reagieren erstere bevorzugt und schneller mit Halogenozinkcarbonylverbindungen der allgemeinen Formel (3) (Reformatsky-Reagenzien), wobei Hal Halogenozink bedeutet, als die als Lösungsmittel verwendeten Carbonsäureester der allgemeinen Formel (4).

Legt man daher zuerst das Zink in dem Carbonsäureester der allgemeinen Formel (4) vor, gibt anschließend die reaktive Halogenverbindung der allgemeinen Formel (3) zu und fügt zuletzt das Elektrophil zu, dann verläuft die Umsetzung unter Bildung von unerwünschten Nebenprodukten. Deshalb wurden Carbonsäureester der allgemeinen Formel (4) bisher nicht als Lösungsmittel für die Umsetzung von Aldehyden, Ketonen und Iminen mit reaktiven Halogenverbindungen und Zink und zur Lagerung von Halogenozinkcarbonylverbindungen der allgemeinen Formel (3) (Reformatsky-Reagenzien), wobei Hal Halogenozink bedeutet, eingesetzt.

Die C₁-C₃₀-Kohlenwasserstoffreste für **R**^{**1**}**, R**^{**2**}**, R**^{**3**}**, R**^{**4**} und **Z** sind vorzugsweise lineare, verzweigte oder cyclische C₁-C₂₀-Alkyl-, C₂-C₂₀-Alkenyl-, C₅-C₂₀-Acetalalkenyl-, C₃-C₂₀-Alkoxycarbonylalkylreste, die substituiert sein können durch F, Cl, Br, J, CN und C₁-C₈-Alkoxyreste; Aryl-, Aralkyl-, Alkaryl-, Aralkenyl-, Alkenylarylreste, in denen eine oder mehrere Methineinheiten durch Gruppen -N= oder -P=, und Methyleneinheiten durch -O-, -S-, -NH- ersetzt sein können und die substituiert sein können durch F, Cl, Br, J, CN, und C₁-C₁₀-Alkoxyreste und am Ring durch C₁-C₁₀-Alkylreste und die im Ring die Heteroatome -O-, -S- oder -NH- tragen können. Die Reste **R**^{**1**} und **R**^{**2**}**, R**^{**3**} und **R**^{**4**}**, R**^{**1**} und **R**^{**3**}**, R**^{**1**} und **Y, R**^{**1**} und **Z, R**^{**3**} und **Y, R**^{**3**} und **Z, W**^{**x**} und **Y** sowie **W**^{**x**} und **Z,** wobei **W**^{**x**} O- oder NR¹- bedeutet und **Z** eine direkte Bindung bedeuten kann, können miteinander verknüpft sein. Die Reste **R**^{**1**} und **R**^{**3**}**, R**^{**1**} und **Y, R**^{**1**} und **Z, W**^{**x**} und **Y** sowie **W**^{**x**} und **Z,** wobei **W**^{**x**} O- oder NR¹- bedeutet und **Z** eine direkte Bindung bedeuten kann, können durch intramolekulare Reaktion miteinander verknüpft sein.

Die Halogenreste **R**^{**3**} und **R**^{**4**} sind vorzugsweise F und Cl. Insbesondere bedeutet **1** den Wert 0.

Als reaktive Halogenverbindungen der allgemeinen Formel (3) werden Bromverbindungen, wobei Hal in der allgemeinen Formel (3) Brom bedeutet, bevorzugt eingesetzt. Bevorzugt sind reaktive Halogenverbindungen der allgemeinen Formel (3), bei der **Y** die Bedeutung **(C=O)-Z** hat. Weiterhin sind reaktive Halogenverbindungen der allgemeinen Formel (3) bevorzugt, bei denen **Z** die Bedeutung **OR**^{**1**} hat. Insbesondere sind als reaktive Halogenverbindungen der allgemeinen Formel (3) α-Bromcarbonsäureester bevorzugt.

Zink wird bevorzugt in Form von Folien, Band-, Span-, Pulver-oder Staubform oder in Form von Zinkwolle eingesetzt. Die Anwesenheit andere Metalle wie Kupfer, Silber oder Quecksilber ist nicht erforderlich. Insbesondere wird Zink in Form von kommerziell erhältlichem, handelsüblichem Zinkpulver oder Zinkstaub eingesetzt.

Zur Erzielung hoher Produktausbeuten hat es sich bewährt, das Zink vor Zugabe des Aldehyds, Ketons oder Imins der allgemeinen Formel (2) und der reaktiven Halogenverbindung der allgemeinen Formel (3) bzw. deren Mischung zu aktivieren. Zur Zinkaktivierung sind vorbekannte, beispielsweise in der zusammenfassenden Darstellung von A. Fürstner, Synthesis 1989, 571, genannte, üblicherweise verwendete Methoden geeignet. Bewährt haben sich die Wäsche des Zinks mit Säure, die Aktivierung durch Iod wie in EP-A-562 343 beschrieben und die Aktivierung durch Trimethylchlorsilan, wobei die Aktivierung durch Trimethylchlorsilan aufgrund ihrer einfachen Durchführbarkeit und der gesteigerten Ausbeuten, Produktreinheiten und Selektivitäten sowie der Unterdrückung von Nebenreaktionen besonders bevorzugt ist. Aus G. Picotin, P. Miginiac, J. Org. Chem. 1987, 52, 4796, ist die Aktivierung von Zink durch Trimethylchlorsilan in dem Lösungsmittel Diethylether bekannt.

Zur Aktivierung von Zink mit Trimethylchlorsilan wird das Zink in dem Carbonsäureester der allgemeinen Formel (4) vorgelegt, dann Trimethylchlorsilan zugegeben und die Mischung für 10 min bis 2 h, bevorzugt 10 bis 45 min bei Temperaturen von 30 bis 150 °C, insbesondere bei 40 bis 120 °C, bevorzugt bei 50 bis 90 °C erhitzt. Es hat sich bewährt, das Zink mit Trimethylchlorsilan im Molverhältnis 1 : (0.01 bis 0.5), insbesondere 1 : (0.05 bis 0.3) in dem Carbonsäureester der allgemeinen Formel (5) unter Erwärmung auf die gewünschte Temperatur umzusetzen.

Bei den Carbonsäureestern der allgemeinen Formel (4) sind **R**^{**5**} und **R**^{**6**} vorzugsweise geradkettige, verzweigte oder cyclische C₁-C₁₀-Alkyl-, C₆-C₁₀-Aralkyl-, C₂-C₁₀-Alkoxyalkylreste oder C₅-C₁₀-Arylreste. Insbesondere sind **R**^{**5**} und **R**^{**6**} geradkettige oder verzweigte C₁-C₈-Alkylreste.

Vorzugsweise bedeuten **m, n, o, p, q,** und **r** ganze Zahlen im Wert von 0, 1, 2 oder 3. Insbesondere bedeuten **n** und **p** und **r** die Werte 0.

Besonders bevorzugte Carbonsäureester der allgemeinen Formel (4) sind insbesondere Methyl-, Ethyl-, n-Propyl-, i-Propyl-, n-Butyl-, i-Butyl-, t-Butyl-, n-Hexyl-, n-Pentyl-, i-Pentylester der Essigsäure, Propionsäure und Buttersäure und Essigsäurealkyl (C₆-C₁₄) ester-Gemische. Die Ester und die Essigsäurealkyl (C₆-C₁₄) ester-Gemische können bei der Isolierung der hergestellten Produkte in sehr hoher Ausbeute zurückgewonnen und erneut eingesetzt werden. Bei Verwendung des kostengünstigen Methylacetats als Lösungsmittel der allgemeinen Formel (4) kann auf eine Rückgewinnung verzichtet werden.

Zur Aktivierung der Reaktionsmischung können auch Zusatzstoffe wie Kupfer-, Chrom-, Mangan-, Cobalt-, Bismut-, Samarium-, Scandium-, Indium-, Titan-, Cer-, Tellur-, Zinn-, Blei-, Antimon-, Germanium-, Aluminium-, Magnesium-, Palladium-, Nickel- und Quecksilberverbindungen oder gegebenfalls deren Mischungen eingesetzt werden.

Während der Umsetzung im ersten Schritt hält man vorzugsweise die Temperatur der exothermen Reaktion gegebenfalls durch Kühlung auf einem vorgegebenen Wert. Dabei kann die obere Temperaturgrenze durch den Siedepunkt des eingesetzten Lösungsmittels der allgemeinen Formel (4), wie z. B. Ethylacetat (Kp.: 77 °C) oder Essigsäureisopropylester (Kp.: 87-89 °C) begrenzt sein. Bei höhersiedenden Lösungsmitteln der allgemeinen Formel (4), wie z. B. Essigsäure-n-butylester (Kp.: 124-126 °C) wird die Temperatur der Reaktion vorzugsweise durch Kühlung kontrolliert. Die Reaktion wird vorzugsweise bei Temperaturen von -20 bis 150 °C, besonders bevorzugt bei 20 bis 110 °C, insbesondere bei 40 bis 90 °C durchgeführt.

Der Druckbereich der Reaktion ist unkritisch und kann innerhalb weiter Grenzen variiert werden. Der Druck beträgt üblicherweise 0.01 bis 20 bar, bevorzugt wird die Reaktion unter Normaldruck (Atmosphärendruck) durchgeführt.

Die Reaktion wird bevorzugt unter Inertisierung mit Schutzgas, wie Stickstoff oder Argon durchgeführt. Die Reaktion kann kontinuierlich oder diskontinuierlich, bevorzugt diskontinuierlich durchgeführt werden.
Nach Ende der Zugabe aller beteiligten Bestandteile läßt man vorzugsweise noch 5 min bis 3 h, besonders bevorzugt 5 min bis 1.5 h, insbesondere 5 bis 30 min nachreagieren, um die Umsetzung zu vervollständigen.

Überschüssiges Zinkmetall kann durch Filtration abgetrennt werden. Es ist auch möglich, überschüssiges Zink in der im zweiten Schritt zur Hydrolyse der Reaktionsmischung verwendeten Säure aufzulösen.

Es hat sich bewährt, das Zink mit der reaktiven Halogenverbindung der allgemeinen Formel (3) und dem Elektrophil der allgemeinen Formel (2) im Molverhältnis (1 bis 3) : (1 bis 2) : 1, insbesondere (1.1 bis 1.7) : (1 bis 1.3) : 1 in dem Carbonsäureester der allgemeinen Formel (4) als Lösungsmittel umzusetzen.

Darüber hinaus erweist sich das erfindungsgemäße Verfahren gegenüber vorbekannten Verfahrensvarianten als vorteilhaft, da in den meisten Fällen die Nachreaktionszeit mit 5 bis 30 min deutlich verkürzt ist, wodurch insbesondere im technischen Maßstab sehr gute Raum-Zeitausbeuten und damit eine hohe Wirtschaftlichkeit erreicht wird. Sehr kurze Nachreaktionszeiten ergeben sich insbesondere nach Aktivierung der Reaktionsmischung bzw. des Zinks durch Trialkylchlorsilan in dem Carbonsäureester der allgemeinen Formel (4) als Lösungsmittel.

Nach erfolgter Umsetzung im ersten Schritt wird die Reaktionsmischung im zweiten Schritt üblicherweise bei Temperaturen von -30 bis +60 °C, besonders bevorzugt von -10 bis +30 °C durch Zugabe einer wässrigen Säure oder Base hydrolysiert, wobei sich gebildete Zinkverbindungen und Zinksalze auflösen. Alternativ dazu kann man auch das Reaktionsgemisch zu einer wässrigen Säure oder Base zusetzen.

Bevorzugte Basen sind Ammoniak und organische Amine, wie Trialkylamine und Alkanolamine.

Bevorzugte Säuren sind Brönstedt-Säuren, insbesondere starke Säuren, wie Bor-, Tetrafluorobor-, Salpetersäure, salpetrige Säure, Phosphorsäure, phosphorige Säure, unterphosphorige Säure, Schwefelsäure, schwefelige Säure, Peroxoschwefel-, Salz-, Fluß-, Jodwasserstoff-, Bromwasserstoff-, Perchlor-, Hexafluorophosphorsäure, Benzolsulfon-, p-Toluolsulfon-, Methansulfon-, Trifluormethansulfon- und Carbonsäuren, wie Chloressig-, Trichloressig-, Essig-, Acryl-, Benzoe-, Trifluoressig-, Citronen-, Croton-, Ameisen-, Fumar-, Malein-, Malon-, Gallus-, Itacon-, Milch-, Wein-, Oxal-, Phthal- und Bernsteinsäure.

Insbesondere wird Ammoniak, Salzsäure, Schwefelsäure oder Citronensäure, bevorzugt Ammoniak, Salzsäure und Citronensäure eingesetzt. Die Säure oder Base kann in konzentrierter Form oder in Form einer verdünnten wässrigen Lösung eingesetzt werden.

Die hergestellten Produkte der allgemeinen Formel (1) können nach bekannten, üblicherweise verwendeten Methoden wie Extraktion, Destillation, Kristallisation oder mittels chromatographischer Methoden isoliert werden. In den meisten Fällen ist das nach Entfernung des Lösungsmittels erhaltene Rohprodukt von sehr hoher bzw. ausreichender Reinheit und kann unmittelbar in nachfolgenden Reaktionen und Umsetzungen, insbesondere Esterhydrolysen eingesetzt werden.

Alle vorstehenden Symbole der vorstehenden Formeln weisen ihre Bedeutungen jeweils unabhängig voneinander auf.

In den folgenden Beispielen sind, falls jeweils nicht anders angegeben, alle Mengen- und Prozentangaben auf das Gewicht bezogen, alle Drücke 0,10 MPa (abs.) und alle Temperaturen 20°C.

### Beispiel 1:

### Herstellung von 3-Hydroxy-3-methylcapronsäureethylester

Bei Raumtemperatur wurden in einem Dreihalskolben mit Rückflußkühler, Innenthermometer, Tropftrichter und Rührer unter Stickstoff-Schutzgas 11.2 g Zinkpulver (170 mmol) in 65 ml Isopropylacetat vorgelegt. Nachdem 3.2 ml Trimethylchlorsilan (25 mmol) zugefügt wurden, erwärmte man die Mischung 30 min auf 65 °C, ließ dann auf 60 °C abkühlen, fügte 9.7 g Pentan-2-on (113 mmol) unverdünnt zu und tropfte anschließend innerhalb 8 min 22.6 g Bromessigsäureethylester (136 mmol) unverdünnt zu, wobei die Temperatur durch externe Kühlung bei 60 °C gehalten wurde. Dann wurde die Mischung 25 min bei 60 °C gerührt und nach Abkühlung auf 0 °C mit 65 ml 2 N Salzsäure bis zu einem pH-Wert von 1 angesäuert und 30 min gerührt. Überschüssiges Zink wurde anschließend abfiltriert und die organische Phase abgetrennt. Die organische Phase wurde dann bei 0 °C mit 60 ml 1 N Salzsäure gerührt und abschließend mit 30 ml gesättigter Natriumhydrogencarbonat-Lösung gewaschen. Nach Phasentrennung wurde über Natriumsulfat getrocknet und das Lösungsmittel im Vakuum abdestilliert (das Lösungsmittel Isopropylacetat wurde zu > 90 % zurückgewonnen). Nach Destillation wurde 3-Hydroxy-3-methylcapronsäureethylester in einer Ausbeute von 18.2 g (93 % d. Th.) mit einem Siedepunkt von 41 °C (0.31 mbar) erhalten.

Analoge Herstellung in den Lösungsmitteln Ethylacetat, *n*-Butyl-acetat und Methylacetat lieferte 3-Hydroxy-3-methylcapronsäure-ethylester in Ausbeuten von 17.2 g, 16.7 g und 16.2 g (88, 85 bzw. 83 % d. Th.).

### Beispiel 2:

### Herstellung von 3-Hydroxy-3-pbenyl-4-trifluorbuttersäure-methylester

Bei Raumtemperatur wurden in einem Dreihalskolben mit Rückflußkühler, Innenthermometer, Tropftrichter und Rührer unter Stickstoff-Schutzgas 5.7 g Zinkpulver (86 mmol) in 35 ml Ethylacetat vorgelegt. Nachdem 1.63 ml Trimethylchlorsilan (12.8 mmol) zugefügt wurden, erwärmte man die Mischung 30 min auf 65 °C, ließ dann auf 60 °C abkühlen und tropfte anschließend innerhalb 10 min eine Mischung aus 10.6 g Bromessigsäuremethylester (69 mmol) und 10 g ω,ω,ω-Trifluoracetophenon (58 mmol) zu, wobei die Temperatur durch externe Kühlung bei 65 °C gehalten wurde. Dann wurde die Mischung 30 min bei 60 °C gerührt und nach Abkühlung auf 0 °C mit 35 ml 2 N Salzsäure bis zu einem pH-Wert von 1 angesäuert und 30 min gerührt. Überschüssiges Zink wurde anschließend abfiltriert und die organische Phase abgetrennt. Die organische Phase wurde dann bei 0 °C mit 30 ml 0.5 N Salzsäure gerührt und abschließend mit 20 ml gesättigter Natriumhydrogencarbonat-Lösung gewaschen. Nach Phasentrennung wurde über Natriumsulfat getrocknet und das Lösungsmittel im Vakuum abdestilliert. 3-Hydroxy-3-phenyl-4-trifluorbuttersäuremethylester wurde in einer Ausbeute von 13.5 g (94 % d. Th.) erhalten. Die Verbindung besitzt einen Schmelzpunkt von 55 °C (nach Umkristallisation aus Petrolether).

### Beispiel 3:

### Herstellung von 3-Hydroxy-2-methyl-3-phenylbuttersäuremethylester

Bei Raumtemperatur wurden in einem Dreihalskolben mit Rückflußkühler, Innenthermometer, Tropftrichter und Rührer unter Stickstoff-Schutzgas 8.6 g Zinkpulver (130 mmol) in 53 ml Ethylacetat vorgelegt. Nachdem 2.5 ml Trimethylchlorsilan (19.5 mmol) zugefügt wurden, erwärmte man die Mischung 30 min auf 65 °C, ließ dann auf 60 °C abkühlen, fügte 10.5 g Acetophenon (87 mmol) zu und tropfte innerhalb 6 min 17.4 g 2-Brompropionsäuremethylester (104 mmol) zu, wobei die Temperatur durch externe Kühlung bei 65 °C gehalten wurde. Dann wurde die Mischung 25 min bei 60 °C gerührt und nach Abkühlung auf 0 °C mit 70 ml 2 N Salzsäure bis zu einem pH-Wert von 1 angesäuert und 30 min gerührt. Überschüssiges Zink wurde anschließend abfiltriert und die organische Phase abgetrennt. Die organische Phase wurde dann bei 0 °C mit 70 ml 0.5 N Salzsäure gerührt und abschließend mit 30 ml gesättigter Natriumhydrogencarbonat-Lösung gewaschen. Nach Phasentrennung wurde über Natriumsulfat getrocknet und das Lösungsmittel im Vakuum abdestilliert. 3-Hydroxy-2-methyl-3-phenylbuttersäuremethylester wurde in einer Ausbeute von 15.5 g (91 % d. Th.) mit einem Siedepunkt von 64 °C (0.03 mbar) erhalten (das Diastereomerenverhältnis beträgt 2 : 1).

### Beispiel 4:

### Herstellung von 3-Hydroxy-3-(2'-phenylethenyl)capronsäuremethylester

Bei Raumtemperatur wurden in einem Dreihalskolben mit Rückflußkühler, Innenthermometer, Tropftrichter und Rührer unter Stickstoff-Schutzgas 8.5 g Zinkpulver (129 mmol) in 55 ml Ethylacetat vorgelegt. Nachdem 2.4 ml Trimethylchlorsilan (19 mmol) zugefügt wurden, erwärmte man die Mischung 30 min auf 65 °C, ließ dann auf 50 °C abkühlen und tropfte innerhalb 8 min eine Mischung aus 15 g 1-Phenylhex-1-en-3-on (hergestellt durch basenkatalysierte Aldolkondensation von Benzaldehyd und Pentan-2-on) und 15.8 g Bromessigsäuremethylester (103 mmol) zu, wobei die Temperatur durch externe Kühlung bei 50 °C gehalten wurde. Dann wurde die Mischung 30 min bei 50 °C gerührt und nach Abkühlung auf 0 °C mit 60 ml 2 N Salzsäure bis zu einem pH-Wert von 1 angesäuert und 30 min gerührt. Überschüssiges Zink wurde anschließend abfiltriert und die organische Phase abgetrennt. Die organische Phase wurde dann bei 0 °C mit 60 ml 0.5 N Salzsäure gerührt und abschließend mit 20 ml gesättigter Natriumhydrogencarbonat-Lösung gewaschen. Nach Phasentrennung wurde über Natriumsulfat getrocknet und das Lösungsmittel im Vakuum abdestilliert. 3-Hydroxy-3-(2'-phenylethenyl)capronsäuremethylester wurde in einer Ausbeute von 20.4 g (95 % d. Th.) und einer Reinheit von > 97 % (HPLC) erhalten. Die Verbindung besitzt einen Schmelzpunkt von 43 °C (nach Umkristallisation aus Petrolether).

### Beispiel 5:

### Herstellung von 3-Hydroxy-3-(2'-phenylethyl)capronsäuremethyl-ester

Bei Raumtemperatur wurden in einem Dreihalskolben mit Rückflußkühler, Innenthermometer, Tropftrichter und Rührer unter Stickstoff-Schutzgas 5.6 g Zinkpulver (85 mmol) in 35 ml Ethylacetat vorgelegt. Nachdem 1.61 ml Trimethylchlorsilan (12.6 mmol) zugefügt wurden, erwärmte man die Mischung 30 min auf 65 °C, ließ dann auf 60 °C abkühlen und fügte 10 g 1-Phenylhexan-3-on (hergestellt durch basenkatalysierte Aldolkondensation von Benzaldehyd und Pentan-2-on und anschließender Hydrierung des erhaltenen 1-Phenylhex-1-en-3-on) unverdünnt zu. Anschließend wurden innerhalb 5 min 10.4 g Bromessigsäuremethylester (68 mmol) zugetropft, wobei die Temperatur durch externe Kühlung bei 65 °C gehalten wurde. Dann wurde die Mischung 30 min bei 60 °C gerührt und nach Abkühlung auf 0 °C mit 35 ml 2 N Salzsäure bis zu einem pH-Wert von 1 angesäuert und 30 min gerührt. Überschüssiges Zink wurde anschließend abfiltriert und die organische Phase abgetrennt. Die organische Phase wurde dann bei 0 °C mit 30 ml 0.5 N Salzsäure gerührt und abschließend mit 20 ml gesättigter Natriumhydrogencarbonat-Lösung gewaschen. Nach Phasentrennung wurde über Natriumsulfat getrocknet und das Lösungsmittel im Vakuum abdestilliert. 3-Hydroxy-3-(2'-phenylethyl)capronsäure-methylester wurde in einer Ausbeute von 13.5 g (95 % d. Th.) und einer Reinheit von > 97 % (HPLC) erhalten.

### Beispiel 6:

### Herstellung von 3-Hydroxy-3-phenylpropionsäuremethylester

Bei Raumtemperatur wurden in einem Dreihalskolben mit Rückflußkühler, Innenthermometer, Tropftrichter und Rührer unter Stickstoff-Schutzgas 5.7 g Zinkpulver (87 mmol) in 35 ml Isopropylacetat vorgelegt. Nachdem 1.65 ml Trimethylchlorsilan (13 mmol) zugefügt wurden, erwärmte man die Mischung 30 min auf 65 °C, ließ dann auf 60 °C abkühlen, fügte 6.2 g Benzaldehyd (58 mmol) zu und tropfte innerhalb 4 min bei 55 °C 10.6 g Bromessigsäuremethylester (70 mmol) zu, wobei die Temperatur durch externe Kühlung bei 55 °C gehalten wurde. Dann wurde die Mischung 5 min bei 55 °C gerührt und nach Abkühlung auf 0 °C mit 40 ml 2 N Salzsäure bis zu einem pH-Wert von 1 angesäuert und 30 min gerührt. Überschüssiges Zink wurde anschließend abfiltriert und die organische Phase abgetrennt. Die organische Phase wurde dann bei 0 °C mit 40 ml 0.5 N Salzsäure gerührt und abschließend mit 20 ml gesättigter Natriumhydrogencarbonat-Lösung gewaschen. Nach Phasentrennung wurde über Natriumsulfat getrocknet und das Lösungsmittel im Vakuum abdestilliert. 3-Hydroxy-3-phenylpropionsäuremethylester wurde in einer Ausbeute von 8.9 g (85 % d. Th.) mit einem Siedepunkt von 76 °C (0.07 mbar) erhalten.

Analoge Herstellung im Lösungsmittel Ethylacetat lieferte 3-Hydroxy-3-phenylpropionsäuremethylester in einer Ausbeute von 9.1 g (87 % d. Th.).

### Beispiel 7:

### Herstellung von 3-Hydroxydecansäure

Bei Raumtemperatur wurden in einem Dreihalskolben mit Rückflußkühler, Innenthermometer, Tropftrichter und Rührer unter Stickstoff-Schutzgas 11.1 g Zinkpulver (169 mmol) in 70 ml Ethylacetat vorgelegt. Nachdem 3.2 ml Trimethylchlorsilan (25 mmol) zugefügt wurden, erwärmte man die Mischung 30 min auf 65 °C, ließ dann auf 60 °C abkühlen und tropfte anschließend innerhalb 6 min eine Mischung aus 21 g Bromessigsäuremethylester (137 mmol) und 16 g Octanal (125 mmol) zu, wobei die Temperatur durch externe Kühlung bei 65 °C gehalten wurde. Dann wurde die Mischung 5 min bei 55 °C gerührt und nach Abkühlung auf 0 °C mit 75 ml 2 N Salzsäure bis zu einem pH-Wert von 1 angesäuert und 30 min gerührt. Überschüssiges Zink wurde anschließend abfiltriert und die organische Phase abgetrennt. Die organische Phase wurde dann bei 0 °C mit 80 ml 1 N Salzsäure gerührt und abschließend mit 80 ml gesättigter Natriumhydrogencarbonat-Lösung gewaschen.

Nach Phasentrennung wurde das Lösungsmittel im Vakuum abdestilliert. Das Rohprodukt wurde zur Esterhydrolyse mit einer Lösung von 15.4 g Kaliumhydroxid (232 mmol) in 140 ml Wasser versetzt und 40 min bei 50 °C gerührt, wobei sich eine klare Lösung bildete. Es wurde zweimal mit je 20 ml Toluol extrahiert, die wäßrige Phase bei 0 °C mit 100 ml 2 N Salzsäure bis zu einem pH-Wert von 1 angesäuert und anschließend dreimal mit je 20 ml Ethylacetat extrahiert. Die organischen Extrakte wurden über Natriumsulfat getrocknet und das Lösungsmittel im Vakuum abdestilliert. 3-Hydroxydecansäure wurde in einer Ausbeute von 21 g (89 % d. Th.) erhalten. Die Verbindung besitzt einen Schmelzpunkt von 58 °C (nach Umkristallisation aus Petrolether).

### Beispiel 8:

### Herstellung von 3-Hydroxy-2-methyldecansäuremethylester

Bei Raumtemperatur wurden in einem Dreihalskolben mit Rückflußkühler, Innenthermometer, Tropftrichter und Rührer unter Stickstoff-Schutzgas 5.5 g Zinkpulver (84 mmol) in 34 ml Ethylacetat vorgelegt. Nachdem 1.6 ml Trimethylchlorsilan (12.5 mmol) zugefügt wurden, erwärmte man die Mischung 30 min auf 65 °C, ließ dann auf 60 °C abkühlen, fügte 7.2 g Octanal (56 mmol) zu und tropfte innerhalb 4 min 11.2 g 2-Brompropionsäuremethylester (67 mmol) zu, wobei die Temperatur durch externe Kühlung bei 65 °C gehalten wurde. Dann wurde die Mischung 5 min bei 60 °C gerührt und nach Abkühlung auf 0 °C mit 40 ml 2 N Salzsäure bis zu einem pH-Wert von 1 angesäuert und 30 min gerührt. Überschüssiges Zink wurde anschließend abfiltriert und die organische Phase abgetrennt. Die organische Phase wurde dann bei 0 °C mit 40 ml 0.5 N Salzsäure gerührt und abschließend mit 20 ml gesättigter Natriumhydrogencarbonat-Lösung gewaschen. Nach Phasentrennung wurde über Natriumsulfat getrocknet und das Lösungsmittel im Vakuum abdestilliert. 3-Hydroxy-2-methyldecansäuremethylester wurde in einer Ausbeute von 10.2 g (85 % d. Th.) mit einem Siedepunkt von 68 °C (0.04 mbar) erhalten (das Diastereomerenverhältnis beträgt 2 : 1).

### Beispiel 9:

### Herstellung von N-Phenyl-3-phenylacetidin-2-on.

Bei Raumtemperatur wurden in einem Dreihalskolben mit Rückflußkühler, Innenthermometer, Tropftrichter und Rührer unter Stickstoff-Schutzgas 7.6 g Zinkpulver (115 mmol) in 45 ml Ethylacetat vorgelegt. Nachdem 1.8 ml Trimethylchlorsilan (14 mmol) zugefügt wurden, erwärmte man die Mischung 30 min auf 70 °C und tropfte dann innerhalb 8 min eine Mischung aus 16.1 g Benzalanilin (89 mmol) und 16.3 g Bromessigsäuremethylester (100 mmol) zu, wobei die Temperatur durch externe Kühlung bei 75 °C gehalten wurde. Dann wurde die Mischung 30 min bei 60 °C gerührt und nach Abkühlung auf 10 °C zuerst mit 130 ml Ethylacetat versetzt und anschließend mit 80 ml 25 %-iger Ammoniak-Lösung hydrolysiert. Die Mischung wurde dann 20 min bei 55 °C gerührt. Überschüssiges Zink wurde anschließend in der Wärme abfiltriert und die organische Phase abgetrennt. Die organische Phase wurde dann in der Wärme mit 20 ml Wasser gewaschen. Nach Phasentrennung wurde das Lösungsmittel im Vakuum abdestilliert, wobei ein Rohprodukt, das zu 91.7 Mol-% aus N-Phenyl-3-phenylacetidin-2-on (β-Lactam) und zu 8.3 Gew.-% aus 3-(N-Phenylamino)-3-phenylpropionsäuremethylester (β-Aminosäureester) bestand, in einer Ausbeute von 19.4 g (97 % d. Th.) und einer Reinheit von > 95 % (HPLC) erhalten wurde. Nach Umkristallisation aus Ethylacetat wurde N-Phenyl-3-phenylacetidin-2-on mit einem Schmelzpunkt von 156 °C erhalten (nach Umkristallisation aus Methanol beträgt der Schmelzpunkt 153-154 °C: H. Gilman, M. Speeter, J. Am. Chem. Soc. 1943, 65, S. 2255).
Nach A. K. Bose et al., J. Chem. Soc., Chem. Commun. 1984, S. 86 gelingt die Herstellung von N-Phenyl-3-phenylacetidin-2-on in einer Ausbeute von 70 % d.Th..

## Patentansprüche

1. Verfahren zur Herstellung von Hydroxy- und Aminoverbindungen,
bei dem in einem ersten Schritt Aldehyde, Ketone und Imine in einer Reformatsky-Reaktion mit einer reaktiven Halogenverbindung und Zink in Carbonsäureestern als Lösungsmittel umgesetzt werden, wobei die reaktive Halogenverbindung gleichzeitig mit oder nach den Aldehyden, Ketonen und Iminen mit dem Zink in Kontakt gebracht wird und bei dem in einem zweiten Schritt das Reaktionsprodukt des ersten Schritts hydrolysiert wird.

2. Verfahren zur Herstellung von Hydroxy- und Aminoverbindungen der allgemeinen Formel (1)
R¹R²C(W^{x})-R³R⁴C-(X)₁-Y (1),
bei dem in einem ersten Schritt Aldehyde, Ketone und Imine der allgemeinen Formel (2)
R¹R²C=W (2)
mit reaktiven Halogenverbindungen der allgemeinen Formel (3)
Hal-R³R⁴C-(X)₁-Y (3),
und Zink in Carbonsäureestern der allgemeinen Formel (4) als Lösungsmittel
R⁵-((O(CH₂)ₘ)ₙ-COO-((CH₂)ₒ-COO)ₚ-((CH₂)_{q}O)ᵣ-R⁶ (4),
umgesetzt werden, wobei
**R**^{**1**} und **R**^{**2**} Wasserstoff oder einen gegebenenfalls halogen- oder cyanosubstituierten C₁-C₃₀-Kohlenwasserstoffrest, in dem eine oder mehrere, einander nicht benachbarte Methyleneinheiten durch Gruppen -O-, -CO-, -COO-, -OCO-, oder -OCOO-, -S- oder -N**R**^{**x**}- ersetzt sein können und in dem eine oder mehrere Methineinheiten durch Gruppen -N= oder - P= ersetzt sein können,
**W**^{**x**} OH oder NHR¹,
**W** O oder NR¹,
**R**^{**3**} und **R**^{**4**} Wasserstoff, Halogen oder einen gegebenenfalls halogensubstituierten C₁-C₃₀-Kohlenwasserstoffrest, in dem eine oder mehrere, einander nicht benachbarte Methyleneinheiten durch Gruppen -O-, -CO-, -COO-, -OCO-, oder -OCOO-, -S- oder -N**R**^{**x**}- ersetzt sein können und in dem eine oder mehrere Methineinheiten durch Gruppen -N= oder - P= ersetzt sein können,
**X** ausgewählt wird aus 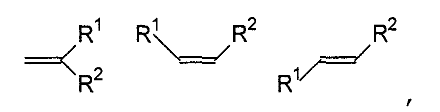
**l** eine ganze Zahl im Wert von 0 oder 1,
**Y** CN, (C=O)-Z, (SO₂)-Z, (P=O) (-Z)₂ oder ein aromatischer Rest, wobei eine oder mehrere Methineinheiten im Ring durch Gruppen -N= oder -P= ersetzt sein können und der Ring die Heteroatome -O-, -S- oder -NH- tragen kann, wobei der aromatische Ring gegebenenfalls halogen- oder cyanosubstituiert oder durch C₁-C₃₀-Kohlenwasserstoffreste, in denen eine oder mehrere, einander nicht benachbarte Methyleneinheiten durch Gruppen -O-, -CO-, -COO-, -OCO-, oder -OCOO-, -S- oder -N**R**^{**x**}- ersetzt sein können, substituiert ist,
**Z** einen gegebenenfalls halogensubstituierten C₁-C₃₀-Kohlenwasserstoffrest, in dem eine oder mehrere, einander nicht benachbarte Methyleneinheiten durch Gruppen -O-, -CO-, -COO-, -OCO-, oder -OCOO-, -S-, oder -N**R**^{**x**}- ersetzt sein können und in dem eine oder mehrere Methineinheiten durch Gruppen -N= oder -P= ersetzt sein können, OH, OR¹, OSi(R¹)₃, NHR¹ oder NR¹R²,
**Hal** Chlor, Brom oder Iod,
**R**^{**5**} und **R**^{**6**} einen C₁-C₃₀-Kohlenwasserstoffrest, in dem eine oder mehrere, einander nicht benachbarte Methyleneinheiten durch Gruppen -O- ersetzt sein können,
**R**^{**x**} Wasserstoff oder einen gegebenenfalls halogensubstituierten C₁-C₃₀-Kohlenwasserstoffrest, in dem eine oder mehrere, einander nicht benachbarte Methyleneinheiten durch Gruppen -O-, -CO-, -COO-, -OCO-, oder -OCOO-, -S-, -NH- oder -NCH₃-ersetzt sein können und in dem eine oder mehrere Methineinheiten durch Gruppen -N= oder -P= ersetzt sein können,
**m, n, o, p, q,** und **r** ganze Zahlen im Wert von 0 bis 6 bedeuten, wobei jeweils 2 Reste, die ausgewählt werden aus den Paaren **R**^{**1**} und **R**^{**2**}**, R**^{**3**} und **R**^{**4**}**, R**^{**1**} und **R**^{**3**}**, R**^{**1**} und **Y, R**^{**1**} und **Z, R**^{**3**} und **Y, R**^{**3**} und **Z, W**^{**x**} und **Y** sowie **W**^{**x**} und **Z,** wobei **W**^{**x**} O- oder NR¹-bedeutet und **Z** eine direkte Bindung bedeuten kann, miteinander verknüpft sein können,
wobei die reaktiven Halogenverbindungen der allgemeinen Formel (3) gleichzeitig mit oder nach den Verbindungen der allgemeinen Formel (2) mit dem Zink in Kontakt gebracht werden und
bei dem in einem zweiten Schritt das Reaktionsprodukt des ersten Schritts hydrolysiert wird.

3. Verfahren nach Anspruch 1 oder 2, bei dem als reaktive Halogenverbindungen Bromverbindungen eingesetzt werden.

4. Verfahren nach Anspruch 2 oder 3, bei dem bei den reaktiven Halogenverbindungen der allgemeinen Formel (3) **Y** die Bedeutung **(C=O)-Z** hat.

5. Verfahren nach Anspruch 4, bei dem **Z** die Bedeutung **OR**^{**1**} hat.

6. Verfahren nach Anspruch 1 bis 5, bei dem als reaktive Halogenverbindungen α-Bromcarbonsäureester eingesetzt werden.

7. Verfahren nach Anspruch 1 bis 6, bei dem der Carbonsäureester ausgewählt wird aus Methyl-, Ethyl-, n-Propyl-, i-Propyl-, n-Butyl-, i-Butyl-, t-Butyl-, n-Hexyl-, n-Pentyl-, i-Pentylacetat, -propionat und -butyrat und Essigsäurealkyl(C₆-C₁₄)ester-Gemischen.

8. Verfahren nach Anspruch 1 bis 7, bei dem das eingesetzte Zink mit Trimethylchlorsilan aktiviert wird.
